# FASCICULE DE BREVET EUROPEEN

(11) **EP 2 819 979 B1**
(45) Date de publication et mention de la délivrance du brevet: **01.08.2018**
(21) Numéro de dépôt: 13712834.4
(22) Date de dépôt: 22.02.2013
(51) Int. Cl.: C07C 17/23, C07C 17/383, C07C 21/18, B01J 23/44, H01L 41/193, B01J 35/10, H01L 41/45

(54) **PROCÉDÉ DE SYNTHÈSE DU TRIFLUOROÉTHYLÈNE À PARTIR DU CHLOROTRIFLUOROÉTHYLÈNE**
VERFAHREN ZUR SYNTHESE VON TRIFLUORETHYLEN AUS CHLORTRIFLUORETHYLEN
METHOD FOR SYNTHESISING TRIFLUOROETHYLENE FROM CHLOROTRIFLUOROETHYLENE

(30) Priorité: 28.02.2012 FR 1251784
(43) Date de publication de la demande: 07.01.2015
(73) Titulaire: Arkema France, 92700 Colombes (FR)
(72) Inventeur: LEDUC, Philippe, F-69590 Larajasse (FR); LANNUZEL, Thierry, F-69100 Villeurbanne (FR); GARRAIT, Dominique, F-69390 Charly (FR); HUB, Serge, F-69100 Villeurbanne (FR); GUIRAUD, Emmanuel, F-69230 Saint-genis Laval (FR); DOMINGUES DOS SANTOS, Fabrice, 75003 PARIS (FR)
(74) Mandataire: Albani, Dalila
(86) Numéro de dépôt international: PCT/FR2013/050373
(87) Numéro de publication internationale: WO 2013/128102

(56) Documents cités:
- US-A- 3 564 064
- US-A1- 2008 081 195

## Description

### Domaine de l'invention

La présente invention concerne le domaine des hydrocarbures fluorés insaturés et a plus particulièrement pour objet la préparation du trifluoroéthylène (VF₃ ou TrFE) par hydrogénolyse du chlorotrifluoroéthylène en phase gazeuse sur un catalyseur d'un métal du groupe VIII déposé sur un support.

### Arrière-plan technique

Les oléfines fluorées, comme le VF₃, sont connues et sont utilisées comme monomères ou co-monomères pour la fabrication de polymères fluorocarbonés présentant des caractéristiques remarquables, en particulier une excellente tenue chimique et une bonne résistance thermique.

Le trifluoroéthylène est un gaz dans les conditions normales de pression et de température. Les principaux risques liés à l'utilisation de ce produit concernent son inflammabilité, sa propension à l'auto-polymérisation lorsqu'il n'est pas stabilisé, son explosivité due à son instabilité chimique et sa supposée sensibilité à la peroxydation, par analogie avec d'autres oléfines halogénées. Le trifluoroéthylène présente la particularité d'être extrêmement inflammable, avec une limite inférieure d'explosivité (LIE) d'environ 10% et une limite supérieure d'explosivité (LSE) d'environ 30%. Le danger majeur est cependant associé à la propension du VF₃ à se décomposer violemment et de façon explosives dans certaines conditions de pression en présence d'une source d'énergie, même en l'absence d'oxygène. Des essais réalisés par la demanderesse pour déterminer la pression limite de stabilité (Pst) du VF₃ (pression maximale pour laquelle il n'y a pas ignition) ont permis de déterminer la Pst du VF₃ à 4 bar. En cas d'explosion, dans les conditions du test, le taux de surpression Pex/Pi est voisin de 10. L'énergie minimale d'ignition est, quant à elle, inconnue. C'est pourquoi il est essentiel d'éviter toute source de chaleur ponctuelle, comme celle résultant de la polymérisation exothermique incontrôlée (auto-polymérisation). Enfin, le VF₃ étant un éthylénique halogéné, il fait partie des composés peroxydables. Le risque de peroxydation ainsi que le risque d'auto-polymérisation augmentent en présence de phase liquide. Il y a un risque d'explosion suite à une peroxydation ou à un démarrage de polymérisation lors du stockage de ce type de molécule.

Compte tenu des principaux risques ci-dessus, la synthèse ainsi que le stockage du VF₃ posent des problèmes particuliers et imposent tout au long de ces processus des règles strictes de sécurité.

Plusieurs voies de synthèse du VF₃ sont décrites dans la littérature.

Une première voie, décrite par exemple dans le document EP485246, consiste en l'hydrogénolyse du 1,1,2 - trichloro -1,2,2 - trifluoroéthane (CFC-113) effectuée en phase gazeuse en présence d'un catalyseur mixte à base de cuivre ou d'argent et d'au moins un métal du groupe du platine (ruthénium, rhodium, palladium, osmium, iridium ou platine). Si les matières premières sont facilement disponibles, le produit principal de cette technique est le CTFE, le VF₃ n'étant qu'un sous produit. La durée de vie de ces catalyseurs est relativement courte et il est difficile d'obtenir une bonne sélectivité en VF₃ si on augmente la conversion du CFC 113. Les rendements en VF₃ sont donc faibles.

Une deuxième voie de préparation du trifluoroéthylène repose sur la déshydrofluoration catalytique du tétrafluoroéthane HFC-134a. Le document FR2729136 décrit ainsi un procédé qui emploie du fluorure d'aluminium comme catalyseur. Le trifluoroéthylène est obtenu par déshydrofluoration catalytique en présence de BF₃. Le taux de transformation du HFC-134a est de 18,5%. Les coûts du catalyseur pour cette technique sont faibles, il n'y a pas besoin d'injecter d'hydrogène, ce qui rend la collecte des produits plus simple. Toutefois les conditions opératoires sont difficiles et le taux de transformation du HFC-134a est faible tout comme le rendement en trifluoroéthylène.

Une troisième voie de préparation du trifluoroéthylène est représentée par la réaction de débromation/déchloration du 1,1,2-trifluoro-2-chloro-1-bromoéthane, décrite par exemple dans le document JP57026629. La réaction se fait en présence d'eau et d'un agent de déshalogénation (par exemple Zn). Les conditions réactionnelles sont douces, mais les matières premières sont difficiles à trouver et la réaction produit beaucoup d'effluents.

Une quatrième voie de préparation du trifluoroéthylène, décrite par exemple dans le document US5892135, fait appel à des hydrocarbures halogénés saturés de type CF3CClFX (X étant H, Cl ou F) tels que 124, 114a et 115, en présence d'un catalyseur constitué d'un ou de plusieurs éléments métalliques tels que Ru, Cu, Ni, Cr ou de leurs oxydes ou halogénures métalliques. Le taux de transformation du CF3CClFX peut atteindre 91%, tandis que la sélectivité en trifluoroéthylène peut atteindre 83%, le reste étant du 1132a, HFC-134a et 1122. Cette technique de fabrication de trifluoroéthylène présente un rendement relativement élevé, cependant la température de réaction est élevée (comprise entre 325-425°C), et le catalyseur perd facilement son activité. Elle présente également des difficultés de collecte, de séparation et de purification des produits de réaction.

Une cinquième voie connue de préparation du trifluoroéthylène utilise comme produits de départ le chlorotrifluoroéthylène (CTFE) et l'hydrogène en présence d'un catalyseur ayant comme composants actifs des métaux du groupe VIII et un support constitué de matériaux poreux tels que le charbon actif, l'alumine, l'oxyde de titane, l'oxyde de magnésium, le fluorure de magnésium et le fluorure d'aluminium.

L'hydrogénolyse catalytique de CTFE est généralement réalisée en phase gazeuse.

Par exemple, le document US3564064 décrit un catalyseur à base de Pd ou Pt sur un support de charbon actif ou d'alumine. La température de réaction est comprise entre 200 et 320°C, avec un temps de contact de 0,1 à 4 secondes. Les gaz issus de la réaction sont lavés dans l'eau et une base, puis séchées avec du sulfate de calcium anhydre. Les produits sont récupérés par condensation dans un piège refroidi par un mélange méthanol/carboglace, puis purifiés par distillation fractionnée du mélange récupéré dans le piège refroidi au méthanol/carboglace. Le taux de transformation du CTFE est de plus de 60%, avec une sélectivité en VF₃ de plus de 80%.

L'hydrogénolyse catalytique de CTFE peut cependant se faire en phase liquide en présence d'un accepteur d'acide chlorhydrique et d'un métal du groupe VIII, comme décrit dans le document CN1080277. Le récepteur d'acide est un alcool, une amine, un ester ou un éther; le taux de transformation du CTFE atteint 100%, avec une sélectivité en VF₃ de 80-90% et un rendement de 60-90%. En plus du trifluoroéthylène, on obtient comme sous produits du difluoroéthylène, le 1,1,2-trifluoroéthane et le 1,1-difluoroéthane.

En comparant la technique de production de trifluoroéthylène à partir de CTFE à celle qui part du CFC-113, il y a une diminution des produits de réaction, avec une augmentation relativement importante du rendement en trifluoroéthylène, mais des problèmes de durée de vie du catalyseur et les difficultés dans la collecte, la séparation et la purification évoqués pour les autres voies existent également avec cette technique. Or, ces étapes doivent être compatibles avec une conduite industrielle de la réaction.

Il existe donc un réel besoin de mettre au point un procédé alternatif de préparation de trifluoroéthylène à partir de CTFE, qui permet de pallier les inconvénients précités de sorte à obtenir du VF₃ de manière économique dans des conditions qui limitent au maximum les risques d'explosivité de cette molécule.

Il a maintenant été trouvé que, pour l'hydrogénolyse du CTFE, l'emploi d'un catalyseur à base d'un métal du groupe VIII et plus particulièrement à base de Pd déposé sur un support ainsi qu'une succession particulière d'étapes de séparation et de purification permet, à pression atmosphérique et à des températures peu élevées, d'obtenir d'excellents taux de conversion du CTFE et de sélectivité en VF₃.

### Résumé de l'invention

La présente invention a donc pour objet un procédé de fabrication de trifluoroéthylène (VF₃) à partir de chlorotrifluoroéthylène (CTFE), ledit procédé comprenant les étapes suivantes réalisées à pression atmosphérique :
i) introduire dans un espace de réaction un mélange A constitué d'hydrogène, de CTFE et éventuellement d'un gaz inerte, comme par exemple de l'azote, en phase gazeuse sur un lit de catalyseur à base d'un métal du groupe VIII déposé sur un support, le rapport molaire H₂/CTFE allant de 0,5/1 à 2/1. La température du réacteur est contrôlée par la circulation dans une double enveloppe d'un fluide caloporteur maintenu à une température comprise entre 25 et 50°C conduisant après un temps de contact suffisant à l'obtention d'un mélange gazeux B constitué des produits de réaction comprenant du VF₃ et des sous-produits organiques, ainsi que de l'H₂, du gaz inerte éventuel et du CTFE non consommés et des hydracides ;
ii) éliminer les hydracides présents dans le mélange B par lavage à l'eau suivi d'un lavage avec une base diluée puis séchage, conduisant à la récupération d'un mélange gazeux C constitué de produits de réaction comprenant du VF₃ et des sous-produits organiques, ainsi que de l'H₂, du gaz inerte (si présent) et du CTFE non consommés ;
iii) passer le mélange gazeux C dans une colonne à contrecourant d'un solvant, à une température inférieure à la température ambiante, conduisant à l'obtention d'une part, de l'hydrogène et du gaz inerte (si présent) et d'autre part, d'un mélange constitué de produits organiques dissouts dans ledit solvant;
iv) désorption, par chauffage à l'ébullition, des produits organiques dissouts dans le solvant afin d'obtenir d'une part le solvant, qui sera recyclé à l'absorption, et d'autre part un mélange D constitué des produits de la réaction exempts d'hydrogène et d'autres gaz inertes (si présents) ;
v) distiller ledit mélange D de produits organiques sur une première colonne, conduisant à la récupération du VF₃ en tête de colonne, et d'un mélange E en pied de colonne constitué de CTFE non converti ainsi que des sous produits de la réaction ;
vi) distiller ledit mélange E afin de récupérer et recycler le CTFE non converti en tête de colonne et d'éliminer les sous produits de la réaction en pied de cette deuxième colonne.

Le procédé faisant l'objet de la présente invention présente l'avantage d'atteindre une conversion très élevée en VF₃ (jusqu'à 80%), avec une sélectivité en VF₃ allant jusqu'à 80% tout en limitant au maximum les risques d'explosivité de cette molécule et en réduisant considérablement la température de réaction, de sorte à pouvoir l'appliquer efficacement à l'échelle industrielle.

D'autres caractéristiques et avantages ressortiront de la description détaillée du procédé de fabrication de VF₃ à partir de CTFE selon l'invention qui va suivre et des figures annexées dans lesquelles :
- la figure 1 illustre de manière schématique l'ensemble des étapes de fabrication du VF₃ à partir du CTFE ;
- la figure 2 illustre le schéma réactionnel proposé pour expliquer la présence de sous-produits identifiés par analyse chromatographique.

### Description détaillée de l'invention

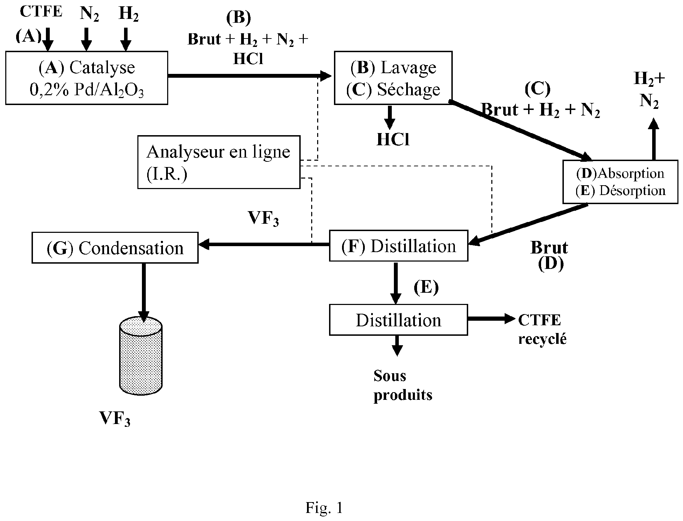

Le procédé selon l'invention, représenté schématiquement dans la figure 1 annexée, est basé sur la réaction de synthèse du VF₃ par hydrogénolyse du CTFE. La réaction est réalisée sur un catalyseur à base d'un métal du groupe VIII déposé sur un support. Les métaux du groupe VIII qui conviennent pour cette réaction sont choisis parmi : Pd, Pt, Rh et Ru. Le support du catalyseur est choisi parmi le charbon actif, l'alumine et le carbonate de calcium.

Selon un mode de réalisation, la réaction est effectuée sur un catalyseur à base de palladium déposé sur Al₂O₃ tel que celui commercialisé par Johnson Matthey sous la référence : 0.2R463 Palladium on Alumina pellets Type 463.

Le procédé consiste à introduire simultanément de l'hydrogène, le CTFE et optionnellement un gaz inerte, comme l'azote (formant le mélange A) en phase gazeuse sur un lit de catalyseur dont la température est contrôlée par circulation dans la double enveloppe du réacteur d'un fluide caloporteur maintenu entre 25 et 50°C Le rapport molaire H₂/CTFE est compris entre 0,5/1 à 2/1 et de préférence compris entre 1/1 à 1,2/1. Le rapport molaire azote/H2 est compris entre 0/1 à 2/1 et de préférence compris entre 0/1 à 1/1.

Le temps de contact calculé comme étant le rapport entre le volume, en litre, de catalyseur et le débit total du mélange gazeux, en normaux litres par seconde, à l'entrée du réacteur, est compris entre 10 et 30 secondes et de préférence entre 15 et 25 secondes.

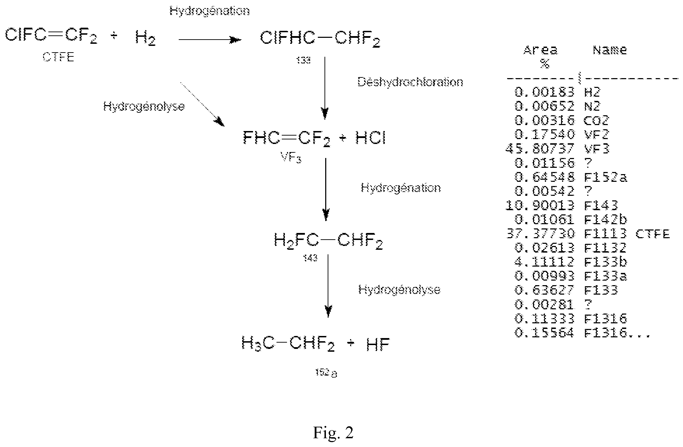

La réaction n'est pas complète, il reste donc de l'hydrogène et du CTFE mélangés avec les produits de réaction et l'azote (si présent) de dilution en sortie du réacteur d'hydrogénolyse. L'ensemble de ces produits forme le mélange B. Le VF₃ est le produit principal de la réaction, mais des sous produits sont formés par des réactions secondaires et/ou successives comme le montre l'analyse chromatographique d'un essai et le schéma réactionnel proposé pour expliquer la formation des ces sous produits (figure 2 annexée).

A la sortie du réacteur d'hydrogénolyse, il est d'abord procédé à l'élimination des hydracides formés. HCl et HF sont absorbés dans de l'eau dans une colonne de lavage les dernières traces d'acide sont éliminées par un lavage avec une base (NaOH ou KOH) diluée. Le reste du mélange gazeux, constitué des réactifs non convertis (H₂ et CTFE), de l'azote de dilution (si présent), et des produits de la réaction (VF₃, 143, 133 et autres produits organiques) qui forment le mélange gazeux C, est dirigé vers un sécheur afin d'éliminer les traces d'eau de lavage. Le séchage est réalisé à l'aide de produits tels que le sulfate de calcium de sodium ou de magnésium, le chlorure de calcium, le carbonate de potassium, le gel de silice (silicagel) ou les zéolites. Dans un mode de réalisation, on utilise pour le séchage un tamis moléculaire (zéolite) tel que la siliporite.

Il est ensuite procédé à une étape de séparation de l'hydrogène et des inertes du reste des autres produits présents dans le mélange C, par absorption/désorption en présence d'un alcool comportant de 1 à 4 atomes de carbone et de préférence l'éthanol, à pression atmosphérique et à une température inférieure la température ambiante, de préférence inférieure à 10°C et de manière encore plus préférée à une température de -25°C, pour l'absorption. Dans un mode de réalisation, l'absorption des organiques est réalisée dans une colonne à contre courant avec de l'éthanol refroidi à -25°C. Le débit d'éthanol est réglé en fonction du débit d'organiques à absorber. L'hydrogène et les gaz inertes, insolubles dans l'éthanol à cette température, sont éliminés en tête de colonne d'absorption. Les organiques sont ensuite récupérés sous forme d'un mélange gazeux D, par chauffage de l'éthanol à son point d'ébullition (désorption), pour être ensuite distillés.

Cette étape de séparation hydrogène + inertes / composés organiques présents dans le mélange C est donc effectuée par une mise en contact direct avec le solvant, à la différence des méthodes de séparation connues pour ce type de mélanges, basées sur la condensation des organiques dans un piège refroidi par un mélange carboglace/solvant (acétone ou méthanol).

Le VF₃ pur est ensuite distillé à partir du mélange D, pour être séparé des autres produits organiques (CTFE, F143, F133 et autres organiques, formant un mélange E). La distillation du mélange D est réalisée à pression atmosphérique et à une température comprise entre - 70°C à la condensation et -30°C au bouilleur. Dans un mode de réalisation, la distillation est du type cryogénique réalisée avec du matériel en verre ; la colonne utilisée est par exemple une colonne du type OLDERSHAW.

Le VF₃ pur sortant en tête de colonne est récupéré par condensation dans des échangeurs et capacités de stockages intermédiaires maintenus à -80°C à pression atmosphérique.

Le mélange E comprenant les autres composés organiques est récupéré en pied de colonne. La distillation dudit mélange E sur une seconde colonne permet de récupérer et recycler le CTFE non converti en tête de colonne et d'éliminer les sous produits de la réaction en pied de cette deuxième colonne.

Le procédé de préparation de VF₃ selon l'invention permet d'obtenir un taux de transformation du CTFE compris entre 60 et 80%, avec une sélectivité en VF₃ comprise entre 50 et 80%.

Le VF₃ pur qui est récupéré dans la capacité de stockage intermédiaire qui sert de recette de distillation est ensuite transféré dans un cylindre de conditionnement. Selon un mode de réalisation, le procédé de l'invention comprend une étape supplémentaire de stockage du VF₃ obtenu à l'étape v) en présence d'une quantité de limonène suffisante pour le stabiliser à une température de maximum de 50°C. Le procédé de préparation du VF₃ par hydrogénolyse du chlorotrifluoroéthylène selon l'invention, réalisé avec l'enchainement en continu des différentes étapes dans des conditions opératoires choisies (pression atmosphérique, basse température), permet de produire du VF₃ tout en évitant les problèmes posés par ses propriétés physicochimiques (inflammabilité, réactivité et explosivité). Cet objectif est atteint en particulier grâce à l'étape de séparation hydrogène et gaz inertes (si présents) des composés organiques par absorption/désorption avec un solvant comme l'éthanol. Les produits de la réaction, ainsi débarrassés des incondensables (hydrogène non converti, inertes...), peuvent ensuite être facilement distillés à pression atmosphérique et à basse température.

D'autres avantages du procédé selon l'invention sont listés ci-dessous:
- il permet de récupérer du VF₃ pur dans des conditions permettant de limiter au
   maximum les risques d'explosivité de cette molécule ;
- il permet de fabriquer du VF₃ de manière économique, dans des conditions telles que les risques d'explosivité de cette molécule, sont évités.
- il permet, en réglant les conditions de distillation, d'obtenir en tête de colonne un mélange de VF₃ et CTFE contenant une proportion déterminée de CTFE qui sera directement utilisable en polymérisation. La présence concomitante éventuelle de 143a, sous-produit de la réaction entrainé en tête de colonne avec le CTFE, n'est pas un inconvénient car ce composé est inerte vis-à-vis de la polymérisation.

Le VF₃ obtenu suivant le procédé de l'invention est utilisé notamment comme monomère ou co-monomère pour la fabrication de polymères fluorocarbonés. Les comonomères susceptibles d'être copolymérisés avec le VF₃ sont notamment les oléfines partiellement ou totalement fluorées. A titre d'exemples non limitatifs, on peut citer le tétrafluoroéthylène, le chlorotrifluoroéthylène (CTFE), l'hexafluoropropylène (HFP), le 1,1,3,3,3-pentafluoropropéne, le fluorure de vinyle, le fluorure de vinylidène (VF₂) et le 2,3,3,3-tetrafluoropropéne. Parmi les autres oléfines halogénées susceptibles d'être copolymérisés avec le VF₂ et le VF₃, on peut citer notamment le CTFE, l'HFP et le 1,1-chlorofluoroéthylène (CFE).

L'invention concerne aussi le procédé de préparation d'un copolymère ou terpolymère fluoré comprenant comme monomère le VF₃ et comme co-monomère au moins une oléfine partiellement ou totalement fluorée, ledit procédé comprenant au préalable la synthèse de VF₃ selon les étapes décrites plus haut.

Selon un mode de réalisation, l'invention concerne un procédé de préparation d'un copolymère ou terpolymère fluoré comprenant comme monomères le VF₃, le CTFE et éventuellement une oléfine partiellement ou totalement fluorée, dans lequel le mélange de VF₃ et de CTFE est obtenu directement en sortie de l'étape de distillation du procédé de synthèse de VF₃ décrit plus haut. L'avantage d'un tel procédé est d'éviter la préparation et l'homogénéisation d'un mélange réalisé à partir des composants purs, simplifiant ainsi les opérations préalables à la polymérisation.

En particulier, le VF₃, lorsqu'il est polymérisé en présence de fluorure de vinylidène et éventuellement d'au moins une autre oléfine halogénée, permet d'obtenir des copolymères ou terpolymères partiellement fluorés électroactifs possédant des propriétés électriques particulièrement intéressantes, tel que le terpolymère poly(VF₃-VF₂-CFE). Les matériaux obtenus à partir de ces copolymères et terpolymères sont alors piézoélectriques, pyroélectriques et/ou électrostrictifs. Les films réalisés à partir de ces copolymères ou terpolymères possèdent les caractéristiques typiques d'un relaxeur ferroélectrique : faible hystérésis, forte permittivité diélectrique, forte déformation.

Des terpolymères VF₃-VF₂-CFE, leur procédé de préparation et leur utilisation pour l'obtention de matériaux composites piézoélectriques sont décrits dans le document US 2008/0081195.

Les exemples suivants illustrent l'invention sans la limiter.

### Exemple 1. Test des performances des catalyseurs

Préparation de la charge catalytique : Le lit de catalyseur à tester est insérer entre deux couches de matériaux inertes tels que le corindon ou la silice. Dans un réacteur tubulaire constitué d'un tube inox d'une longueur de 1200 mm sur un diamètre de 25 mm, et équipé d'une double enveloppe, on introduit dans l'ordre une première couche de corindon sur une hauteur de 50 cm. Sur cette couche on introduit ensuite une couche représentant 100 cm³ de catalyseur à tester puis au dessus de la couche de catalyseur, on introduit à nouveau du corindon jusqu'à remplissage total du tube réactionnel.

Le catalyseur ainsi chargé est ensuite activé sous un flux d'hydrogène de la manière suivante : le tube réactionnel est placé dans un four tubulaire et est alimenté par le haut par un flux d'hydrogène de 2mol/h. On chauffe alors le four jusqu'à 400°C et on laisse sous le même flux d'hydrogène pendant 12h. Après cette période d'activation à 400°C (réduction chimique du catalyseur), le tube est refroidi à température ambiante sous flux d'hydrogène puis est isolé pour être ensuite installer sur un banc de test d'hydrogénolyse.

### Réaction d'hydrogénolyse du CTFE :

Le tube réactionnel chargé du catalyseur activé est installé sur un banc d'hydrogénolyse comprenant :
- une alimentation en CTFE contrôlée par un débit mètre massique ;
- une alimentation en Hydrogène contrôlée par un débit mètre massique ;
- une alimentation en inerte (ici l'azote) contrôlée par un débit mètre massique ;
- une alimentation de la double enveloppe par un fluide caloporteur dont la température est régulée par un bain thermostaté ;
- un système de prélèvement des produits de la réaction permettant d'effectuer les analyses nécessaires au calcul des taux de conversions du CTFE et de l'H₂ d'une part et de la sélectivité en VF₃ d'autre part.

Le tableau n°1 rassemble les conditions opératoires et résultats obtenus avec différents types de charge catalytiques.

Les caractéristiques physico-chimiques des catalyseurs testés sont les suivantes : Catalyseurs sur Al₂O₃ : surface BET environ 5 m²/g et volume poreux < 0,1 cm³/g Catalyseur sur C : surface BET environ 1600 m²/g et volume poreux environ 1 cm³/g.

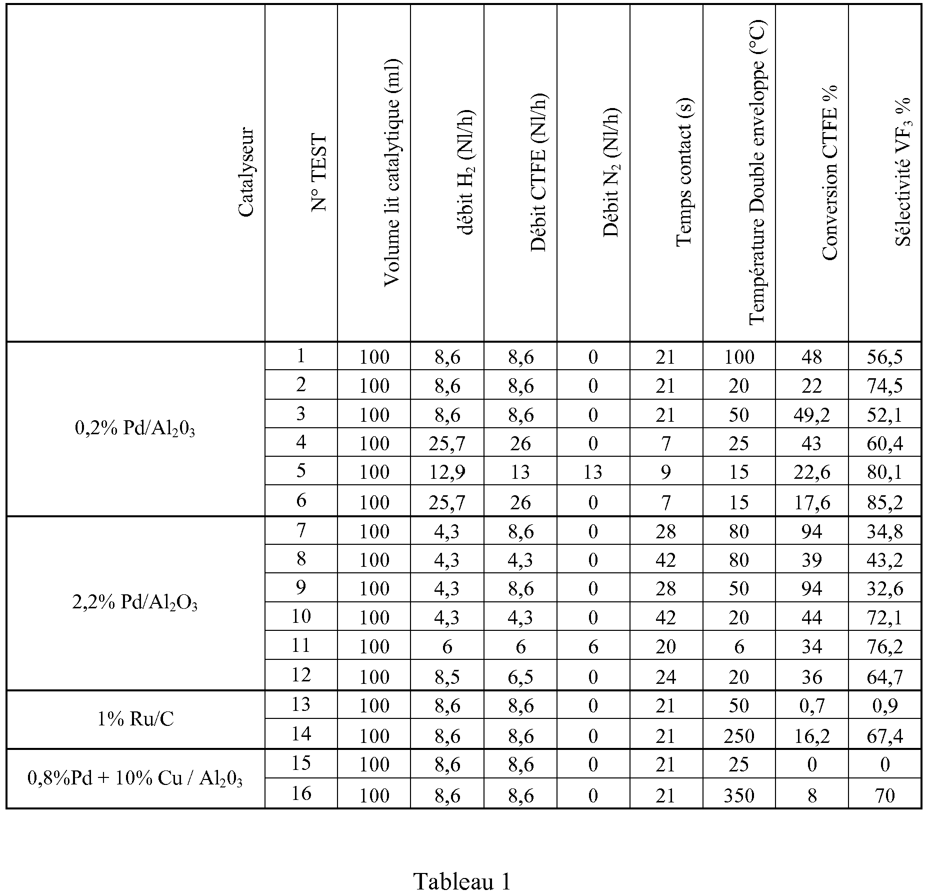

### Exemple 2. Mise en oeuvre du procédé selon l'invention

L'installation micro pilote schématisée en figure 1 a été opérée de la manière suivante :
- On charge 4 réacteurs d'hydrogénolyse (A), constitués chacun d'un tube métallique en inox long de 1200 mm et d'un diamètre de 25 mm, équipé d'une double enveloppe sur toute la longueur du tube, avec le mélange suivant :
   - 66 ml (110 g) de corindon ;
   - 275 ml (436 g) de catalyseur à 0,2% de Pd/Al₂O₃ (surface BET = 5,3 m²/g et un volume poreux < 1 cm/g) ;
   - 45 ml (78 g) de corindon.
- On réduit la composition catalytique à 250°C pendant 6 heures par un débit de 1 mole/h d'hydrogène.
- On alimente ensuite la double enveloppe de chaque réacteur par un fluide caloporteur thermostaté à 25°C.
- On alimente chaque réacteur avec 1mol/h de CTFE et 1mole/h d'hydrogène. Il est également possible d'alimenter les réacteurs avec un gaz inerte (ici de l'azote).

Le temps de contact, calculé comme étant le rapport entre le volume en litre de catalyseur et la somme des débits des réactifs en normaux litres par secondes, est de l'ordre de 22 secondes.

Les gaz sortants des 4 réacteurs sont rassemblés et introduits au bas une colonne d'abattage des hydracides (B) constituée d'un tube en polymère fluoré de 355 mm de long et de 40 mm de diamètre et garni d'anneaux en polymère fluoré de 4 mm de diamètre et de 5 mm de long.

La colonne d'abattage est alimentée en continu par de l'eau à un débit de 101/h.

L'eau chargée en hydracide est éliminée en continu en pied de la colonne d'abattage. Les produits de la réaction ainsi débarrassés des hydracides, sont ensuite dirigés vers une section de séchage (C) constituée de deux tubes métalliques en inox d'une longueur de 800 mm et de 50 mm de diamètre, montés en série, et remplis de tamis moléculaire du type silliporite 3A.

Les gaz ainsi séchés sont ensuite dirigés vers une colonne d'absorption (D) constituée d'un tube métallique en inox de 700 mm de long et de 40 mm de diamètre, équipé d'une double enveloppe et garnie d'anneaux en verre de 4,3 mm de diamètre et de 4,5 mm de long. La colonne d'absorption est alimentée en tête par de l'éthanol via une pompe dont le débit est de 8 litres/heure. La double enveloppe de la colonne d'absorption est alimentée par un fluide caloporteur à -25°C.

L'hydrogène et les inertes sortent en tête de la colonne d'absorption alors que les produits de la réaction, dissouts dans l'éthanol, sortent en pied de la colonne et sont dirigés vers la une section de désorption (E) constituée d'une colonne en verre d'une longueur de 250 mm et de 18 mm de diamètre, garnie d'anneaux de verre de 4,3mm de diamètre et de 4,5 mm de long et d'un ballon en verre de 1 litre ou l'éthanol est porté à l'ébullition grâce à un chauffe ballon.

Les produits organiques issus de la réaction sont évaporés et quittent la section de désorption par la tête de colonne alors que l'éthanol débarrassé des organiques est repris par la pompe pour être alimenté en tête de colonne d'absorption (D).

Le mélange de produits organiques issus de la section de désorption est ensuite dirigé vers la section de distillation (F) constituée d'une section de concentration avec une colonne à 15 plateaux de type OLDERSHAW de 28 mm de diamètre et d'une section d'épuisement avec une colonne à 5 plateaux de type OLDERSHAW de 28 mm de diamètre.

Le bouilleur de cette distillation est constitué d'un réservoir en verre équipé d'une double enveloppe alimentée par un fluide caloporteur à -30°C. Les produits lourds indésirables et le CTFE non converti sont extraits en continu de manière à garder un niveau constant dans le bouilleur.

La colonne de concentration est surmontée d'un réfrigérant en verre alimenté par un fluide caloporteur à -75°C. Le VF₃ pur est récupéré en tête de l'échangeur qui se comporte comme un déflegmateur et est enfin dirigé vers la section de recette (G) constituée de réservoirs métalliques en inox équipés de doubles enveloppes alimentées par un fluide caloporteur maintenu à -80°C.

Le tableau 2 rassemble des résultats de campagnes de production de VF₃ sur ce micro pilote en fonctionnement continu.

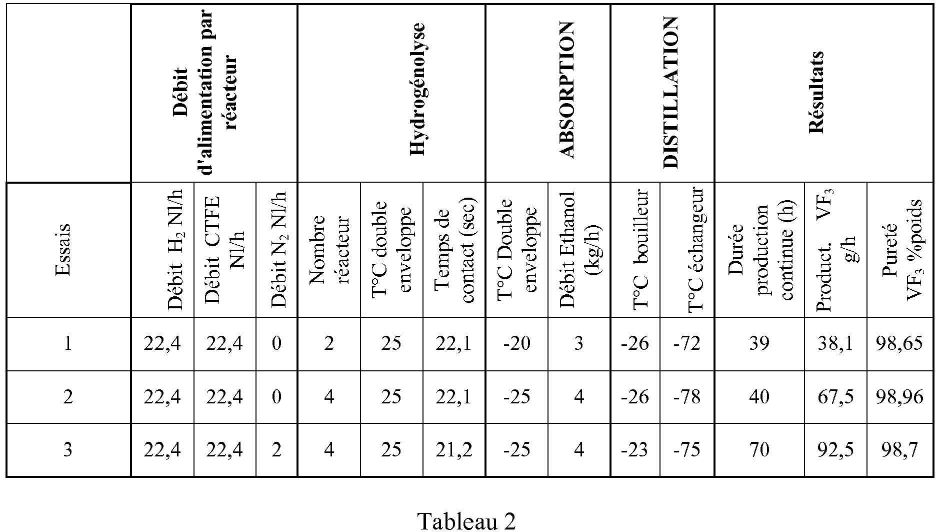

Dans l'essai n°1, seuls deux tubes d'hydrogénolyse sont alimentés en CTFE et Hydrogène. Un débit d'éthanol de la section absorption de 3 kg/h à -20°C suffit à séparer les organiques de l'hydrogène non converti. A la section distillation, une température d'échangeur de -72°C permet d'atteindre une pureté en VF₃ de 98,65%. La productivité en VF₃ est de 38,1g/h ce qui représente un rendement brut de 23,8% par rapport au CTFE introduit.

Dans l'essai n°2, 4 tubes d'hydrogénolyse fonctionnent en parallèle et alimentent le reste de l'installation. Compte tenu du débit d'organiques à traiter, la section absorption est alimentée avec 4kg/h d'éthanol refroidi à -25°C. Dans la section distillation, l'échangeur de tête est alimenté avec un fluide caloporteur à -78°C ce qui permet d'atteindre une pureté du VF₃ de 98,96%. La productivité de l'installation, conduite dans ces conditions est de 67,5g/h de VF₃ soit un rendement brut de 21% par rapport au CTFE alimenté.

Dans l'essai n°3, on alimente les 4 tubes d'hydrogénolyse avec de l'azote en plus des réactifs (CTFE et H2). La présence d'azote ne modifie pas l'efficacité de la séparation de la section absorption alimentée avec 4 kg/h d'éthanol à -25°C. Les conditions de la section distillation sont modifiées de manière à augmenter le taux de récupération du VF₃. Ainsi, avec une température au bouilleur de -23°C et un échangeur de tête à -75°C la productivité est de 92,5g/h de VF₃ avec une pureté de 98,7%. Le recyclage du CTFE non converti permet, dans ces conditions, d'atteindre un rendement brut de 57,8% par rapport au CTFE mis en oeuvre.

### Exemple 3. Préparation d'un copolymère VF₂-VF₃

396 g de VF₃ et 750 g de VF₂ sont chargés dans un réacteur contenant 1862 g d'eau déminéralisée, 0,34 g de methylhydroxypropyl cellulose et 1,8 g de peroxydicarbonate de dipropyle. Le réacteur est alors porté à la température de 44°C. La réaction démarre et se traduit pas une chute de la pression du réacteur qui est compensée par injection continue d'eau. Quand 700 g d'eau ont été introduits, l'injection d'eau est stoppée et la pression chute jusqu'à 65 bars. Le réacteur est alors chauffé à 65°C et la pression continue de chuter. Quand la pression atteint 38 bars, le réacteur est mis à refroidir puis est vidangé. Le mélange réactionnel recueilli est filtré et le gâteau obtenu est lavé à 5 reprises dans 31 d'eau propre avant d'être séché en étuve à 70°C jusqu'à poids constant. La composition de la résine, analysée par RMN 1H, est trouvée égale à 31,2% molaire en VF₃. La caractérisation thermique par DSC révèle une résine ferroélectrique avec une transition de Curie de 99,8°C et une température de fusion de 151,2°C.

### Exemple 4. Préparation d'un terpolymère VF₂-VF₃-CFE

Une charge initiale de 360 g d'un mélange composé de 68% molaire de VF₂ et de 32% molaire de VF₃ est introduite dans un réacteur agité de 4 l chargé de 2,5 l d'eau déminéralisée. Le réacteur est porté à une température de 44°C, puis l'initiateur de polymérisation est injecté par une pompe haute pression sous forme de mélange avec de l'eau. Une quantité supplémentaire d'eau est alors injectée pour amener le réacteur à une pression voisine de 90 b. La réaction de polymérisation démarre et a tendance à faire chuter la pression. Celle-ci est maintenue constante par injection sous pression d'un mélange comprimé secondaire de monomères. Ce mélange a la composition molaire suivante : 58,15 % de VF2, 27,31% de VF3 et 14,54% de CFE. Durant la polymérisation, la température à l'intérieur du réacteur est maintenue à une valeur comprise entre 44 et 51°C. La réaction est poursuivie pendant environ 7h jusqu'à ce que le débit d'injection du mélange secondaire diminue en dessous de 20 g/h. Le polymère, récupéré sous forme d'une poudre solide en suspension dans l'eau, a la composition molaire suivante, évaluée en combinant l'analyse par RMN 19F et le dosage élémentaire de l'élément chlore :
- VF₂ : 61,9 %
- VF₃ : 29,4 %
- CFE : 8,7 %

Sa masse molaire moyenne en nombre est de 350 000 g/mol et sa température de fusion est de 122,1 °C.

### Exemple 5. Préparation d'un terpolymère VF₂-VF₃-CTFE à partir de mélanges VF₃-CTFE provenant directement de la distillation du VF3

Dans un réacteur agité de 3,5 l contenant 2,7 l d'eau désionisée et 0,4 g d'ester cellulosique comme agent dispersant, on charge 327 g de VDF, 163 g de TrFE et 10 g CTFE. Les 10 g de CTFE proviennent du TrFE préparé selon l'invention avec un réglage des conditions de la distillation conduisant à une teneur de 4 % molaire de CTFE dans le TrFE, dosé par chromatographie gazeuse. Le réacteur est alors porté à la température de 46°C. L'initiateur peroxydicarbonate est ensuite injecté et la réaction démarre. La consommation des monomères induit une diminution de la pression qui est compensée par l'injection continue d'un mélange VDF-TrFE-CTFE à une pression comprise entre 80 et 110 bars. Le mélange, dont la composition molaire est 67/26/1 respectivement, a été préparé à partir de VDF pur et de TrFE contenant 21% molaire de CTFE, obtenu par le procédé de l'invention et avec un réglage iodine des conditions de distillation. Quand une quantité de 500g de mélange a été introduite dans le réacteur, l'injection est stoppée et on laisse la pression chutée durant 40 minutes. Le réacteur est alors refroidi et dégazé des monomères résiduels, puis le produit, sous forme d'une suspension (slurry), est déchargé du réacteur. Le slurry est filtré, lavé plusieurs fois à l'eau pure désionisée, filtré une dernière fois, puis la poudre humide est mise à sécher en étuve à 70 °C. 720 g de poudre sèche sont récupérés. L'analyse en RMN 1H et 19 F produit révèle la composition molaire suivante :
VF2: 69,8 %
TrFE: 26,5 %
CTFE: 3,7 %.

## Revendications

1. Procédé de fabrication de trifluoroéthylène (VF₃) à partir de chlorotrifluoroéthylène (CTFE), ledit procédé comprenant les étapes suivantes réalisées à pression atmosphérique :
i) introduire dans un réacteur un mélange gazeux A constitué d'hydrogène, de CTFE et éventuellement d'un gaz inerte, sur un lit de catalyseur à base d'un métal du groupe VIII déposé sur un support, le rapport molaire H₂/CTFE allant de 0,5/1 à 2/1, conduisant après un temps de contact suffisant à l'obtention d'un mélange gazeux B constitué des produits de réaction comprenant du VF₃ et des sous-produits organiques, ainsi que de l'H₂, dudit gaz inerte et du CTFE non consommés et des hydracides ;
ii) éliminer les hydracides présents dans le mélange B par lavage à l'eau suivi d'un lavage avec une base diluée puis séchage, conduisant à la récupération d'un mélange gazeux C constitué de produits de réaction comprenant du VF₃ et des sous-produits organiques, ainsi que de l'H₂, dudit gaz inerte et du CTFE non consommés ;
iii) passer le mélange gazeux C dans une colonne à contrecourant d'un solvant, à une température inférieure à la température ambiante, conduisant à l'obtention d'une part, de l'hydrogène et du gaz inerte et d'autre part, d'un mélange constitué de produits organiques dissouts dans ledit solvant;
iv) désorption, par chauffage à l'ébullition, des produits organiques dissouts dans le solvant afin d'obtenir d'une part le solvant, qui sera recyclé à l'absorption, et d'autre part un mélange D constitué des produits de la réaction exempts d'hydrogène et de gaz inerte ;
v) distiller ledit mélange D de produits organiques, conduisant à la récupération du VF₃ en tête de colonne, et d'un mélange E en pied de colonne constitué de CTFE non converti ainsi que des sous produits de la réaction.

2. Procédé selon la revendication 1 comprenant une étape supplémentaire vi) de distillation dudit mélange E sur une deuxième colonne afin de récupérer et recycler le CTFE non converti en tête de colonne et d'éliminer les sous produits de la réaction en pied de cette deuxième colonne.

3. Procédé selon l'une des revendications 1 et 2 comprenant une étape supplémentaire de stockage du VF₃ obtenu à l'étape v) en présence d'une quantité de limonène suffisante pour le stabiliser à une température de maximum de 50°C.

4. Procédé selon l'une des revendications 1 à 3 dans lequel ledit métal est déposé sur un support d'alumine ou de charbon actif.

5. Procédé selon l'une des revendications 1 à 4 dans lequel le catalyseur est à base de Pd déposé sur un support d'alumine.

6. Procédé selon l'une des revendications 1 à 5 dans lequel le solvant utilisé à l'étape iii) est un alcool comportant de 1 à 4 atomes de carbone, de préférence l'éthanol.

7. Procédé selon l'une des revendications 1 à 6 dans lequel le séchage du mélange B après élimination des hydracides se fait sur un tamis moléculaire tel que la siliporite.

8. Procédé selon l'une des revendications 1 à 7 dans lequel ledit gaz inerte est l'azote, le rapport molaire azote/H₂ allant de 0/1 à 2/1 et de préférence de 0/1 à 1/1.

9. Procédé selon l'une des revendications 1 à 8 dans lequel ledit réacteur a une double enveloppe remplie d'un fluide caloporteur dont la température est maintenue entre 25 et 50°C.

10. Procédé de préparation d'un copolymère ou terpolymère fluoré comprenant comme monomère le VF₃ et comme co-monomère au moins une oléfine partiellement ou totalement fluorée, ledit procédé comprenant les étapes suivantes de préparation du VF₃ réalisées à pression atmosphérique:
i) introduire dans un réacteur un mélange gazeux A constitué d'hydrogène, de CTFE et éventuellement d'un gaz inerte, sur un lit de catalyseur à base d'un métal du groupe VIII déposé sur un support, le rapport molaire H₂/CTFE allant de 0,5/1 à 2/1, conduisant après un temps de contact suffisant à l'obtention d'un mélange gazeux B constitué des produits de réaction comprenant du VF₃ et des sous-produits organiques, ainsi que de l'H₂, dudit gaz inerte et du CTFE non consommés et des hydracides ;
ii) éliminer les hydracides présents dans le mélange B par lavage à l'eau suivi d'un lavage avec une base diluée puis séchage, conduisant à la récupération d'un mélange gazeux C constitué de produits de réaction comprenant du VF₃ et des sous-produits organiques, ainsi que de l'H₂, dudit gaz inerte et du CTFE non consommés ;
iii) passer le mélange gazeux C dans une colonne à contrecourant d'un solvant, à une température inférieure à la température ambiante, conduisant à l'obtention d'une part, de l'hydrogène et du gaz inerte et d'autre part, d'un mélange constitué de produits organiques dissouts dans ledit solvant;
iv) désorption, par chauffage à l'ébullition, des produits organiques dissouts dans le solvant afin d'obtenir d'une part le solvant, qui sera recyclé à l'absorption, et d'autre part un mélange D constitué des produits de la réaction exempts d'hydrogène et de gaz inerte ;
v) distiller ledit mélange D de produits organiques, conduisant à la récupération du VF₃ en tête de colonne, et d'un mélange E en pied de colonne constitué de CTFE non converti ainsi que des sous produits de la réaction.

11. Procédé selon la revendication 10, dans lequel ladite oléfine est choisie parmi le tétrafluoroéthylène, le chlorotrifluoroéthylène, l'hexafluoropropylène, le 1,1,3,3,3-pentafluoropropéne, le fluorure de vinyle, le fluorure de vinylidène, le 2,3,3,3-tetrafluoropropéne et le 1,1-chlorofluoroéthylène.

12. Procédé selon l'une des revendications 10 ou 11 conduisant à l'obtention du terpolymère poly(VF₃- VF₂-CFE).

13. Procédé selon la revendication 10, dans lequel ladite oléfine étant le CTFE, le procédé conduit, à la fin de l'étape v), à l'obtention d'un mélange de VF₃ et de CTFE en tête de colonne de distillation.

## Patentansprüche

1. Verfahren zur Herstellung von Trifluorethylen (VF₃) ausgehend von Chlortrifluorethylen (CTFE), wobei das Verfahren die folgenden Schritte umfasst, die bei Atmosphärendruck ausgeführt werden:
i) Einführen eines Gasgemisches A, bestehend aus Wasserstoff, CTFE und gegebenenfalls einem inerten Gas, in einen Reaktor auf ein Katalysatorbett auf Basis eines Metalls der Gruppe VIII, das auf einem Träger abgeschieden ist, wobei das Molverhältnis H₂/CTFE im Bereich von 0,5/1 bis 2/1 liegt, was nach einer ausreichenden Kontaktzeit zur Gewinnung eines Gasgemisches B führt, das aus VF₃ und organische Nebenprodukte umfassenden Reaktionsprodukten sowie H₂, nicht verbrauchtem inertem Gas und CTFE und Wasserstoffsäuren besteht;
ii) Eliminieren der in dem Gemisch B vorhandenen Wasserstoffsäuren durch Waschen mit Wasser, gefolgt von einem Waschen mit einer verdünnten Base und anschließendem Trocknen, was zur Rückgewinnung eines Gasgemisches C führt, das aus VF₃ und organische Nebenprodukte umfassenden Reaktionsprodukten sowie H₂, nicht verbrauchtem inertem Gas und CTFE besteht;
iii) Überführen des Gasgemisches C in eine Säule mit Gegenströmung eines Lösemittels bei einer Temperatur unter der Umgebungstemperatur, was zur Gewinnung einerseits von Wasserstoff und inertem Gas und andererseits eines Gemisches führt, das aus in dem Lösemittel gelösten organischen Produkten besteht;
iv) Desorbieren, durch Erhitzen bis zum Sieden, von in dem Lösemittel gelösten organischen Produkten, um einerseits das Lösemittel, das zur Absorption recycelt wird, und andererseits ein Gemisch D zu gewinnen, das aus den Produkten der Reaktion mit Ausnahme von Wasserstoff und des inerten Gases besteht;
v) Destillieren des Gemisches D von organischen Produkten, was zur Rückgewinnung von VF₃ am Säulenkopf und eines Gemisches E am Säulenfuß führt, das aus nicht umgesetztem CTFE sowie Reaktionsnebenprodukten besteht.

2. Verfahren nach Anspruch 1, umfassend einen zusätzlichen Schritt vi) des Destillierens des Gemisches E in einer zweiten Säule, um das nicht umgesetzte CTFE am Säulenkopf rückzugewinnen und zu recyceln und die Reaktionsnebenprodukte am Fuß dieser zweiten Säule zu eliminieren.

3. Verfahren nach einem der Ansprüche 1 und 2, umfassend einen zusätzlichen Schritt des Lagerns des im Schritt v) gewonnenen VF₃ in Gegenwart einer ausreichenden Menge an Limonen, um es bei einer Temperatur von maximal 50 °C zu stabilisieren.

4. Verfahren nach einem der Ansprüche 1 bis 3, wobei das Metall auf einem Träger aus Aluminiumoxid oder Aktivkohle abgeschieden ist.

5. Verfahren nach einem der Ansprüche 1 bis 4, wobei der Katalysator auf Basis von Pd ist, das auf einem Aluminiumoxid-Träger abgeschieden ist.

6. Verfahren nach einem der Ansprüche 1 bis 5, wobei es sich bei dem im Schritt iii) verwendeten Lösemittel um einen Alkohol handelt, der 1 bis 4 Kohlenstoffatome umfasst, vorzugsweise um Ethanol.

7. Verfahren nach einem der Ansprüche 1 bis 6, wobei das Trocknen des Gemisches B nach dem Eliminieren der Wasserstoffsäuren auf einem Molekularsieb wie Siliporite erfolgt.

8. Verfahren nach einem der Ansprüche 1 bis 7, wobei es sich bei dem inerten Gas um Stickstoff handelt, wobei das Molverhältnis Stickstoff/H₂ von 0/1 bis 2/1 und vorzugsweise von 0/1 bis 1/1 reicht.

9. Verfahren nach einem der Ansprüche 1 bis 8, wobei der Reaktor eine doppelte Ummantelung aufweist, die mit einem Wärmeträgerfluid gefüllt ist, dessen Temperatur zwischen 25 und 50 °C gehalten wird.

10. Verfahren zur Herstellung eines fluorierten Copolymers oder Terpolymers, das als Monomer VF₃ und als Comonomer wenigstens ein teilweise oder vollständig fluoriertes Olefin umfasst, wobei das Verfahren die folgenden Schritte zur Herstellung von VF₃ umfasst, die unter Atmosphärendruck durchgeführt werden:
i) Einführen eines Gasgemisches A, bestehend aus Wasserstoff, CTFE und gegebenenfalls einem inerten Gas, in einen Reaktor auf ein Katalysatorbett auf Basis eines Metalls der Gruppe VIII, das auf einem Träger abgeschieden ist, wobei das Molverhältnis H₂/CTFE im Bereich von 0,5/1 bis 2/1 liegt, was nach einer ausreichenden Kontaktzeit zur Gewinnung eines Gasgemisches B führt, das aus VF₃ und organische Nebenprodukte umfassenden Reaktionsprodukten sowie H₂, nicht verbrauchtem inertem Gas und CTFE und Wasserstoffsäuren besteht;
ii) Eliminieren der in dem Gemisch B vorhandenen Wasserstoffsäuren durch Waschen mit Wasser, gefolgt von einem Waschen mit einer verdünnten Base und anschließendem Trocknen, was zur Rückgewinnung eines Gasgemisches C führt, das aus VF₃ und organische Nebenprodukte umfassenden Reaktionsprodukten sowie H₂, nicht verbrauchtem inertem Gas und CTFE besteht;
iii) Überführen des Gasgemisches C in eine Säule mit Gegenströmung eines Lösemittels bei einer Temperatur unter der Umgebungstemperatur, was zur Gewinnung einerseits von Wasserstoff und inertem Gas und andererseits eines Gemisches führt, das aus in dem Lösemittel gelösten organischen Produkten besteht;
iv) Desorbieren, durch Erhitzen bis zum Sieden, von in dem Lösemittel gelösten organischen Produkten, um einerseits das Lösemittel, das zur Absorption recycelt wird, und andererseits ein Gemisch D zu gewinnen, das aus den Produkten der Reaktion mit Ausnahme von Wasserstoff und des inerten Gases besteht;
v) Destillieren des Gemisches D von organischen Produkten, was zur Rückgewinnung von VF₃ am Säulenkopf und eines Gemisches E am Säulenfuß führt, das aus nicht umgesetztem CTFE sowie Reaktionsnebenprodukten besteht.

11. Verfahren nach Anspruch 10, wobei das Olefin aus Tetrafluorethylen, Chlortrifluorethylen, Hexafluorpropylen, 1,1,3,3,3-Pentafluorpropen, Vinylfluorid, Vinylidenfluorid, 2,3,3,3-Tetrafluorpropen und 1,1-Chlorfluorethylen ausgewählt wird.

12. Verfahren nach einem der Ansprüche 10 oder 11, das zur Gewinnung des Terpolymers Poly(VF₃-VF₂-CFE) führt.

13. Verfahren nach Anspruch 10, wobei es sich bei dem Olefin um CTFE handelt, wobei das Verfahren am Ende des Schritts v) zur Gewinnung eines Gemisches aus VF₃ und CTFE am Kopf der Destillationssäule führt.

## Claims

1. Process for the manufacture of trifluoroethylene (VF₃) from chlorotrifluoroethylene (CTFE), said process comprising the following stages, carried out at atmospheric pressure:
i) introducing, into a reactor, a gas mixture A composed of hydrogen, CTFE and optionally an inert gas over a bed of catalyst based on a metal from Group VIII deposited on a support, the H₂/CTFE molar ratio ranging from 0.5/1 to 2/1, resulting, after a sufficient contact time, in the achievement of a gas mixture B composed of the reaction products comprising VF₃ and organic byproducts, and also of unconsumed H₂, said inert gas and unconsumed CTFE and of hydracids;
ii) removing the hydracids present in the mixture B by washing with water, followed by washing with a dilute base and then drying, resulting in the recovery of a gas mixture C composed of reaction products comprising VF₃ and organic byproducts, and also of unconsumed H₂, said inert gas and unconsumed CTFE;
iii) passing the gas mixture C through a countercurrentwise column of a solvent, at a temperature lower than ambient temperature, resulting in the achievement, on the one hand, of the hydrogen and the inert gas and, on the other hand, of a mixture composed of organic products dissolved in said solvent;
iv) desorbing, by heating to boiling point, the organic products dissolved in the solvent in order to obtain, on the one hand, the solvent, which will be recycled to the absorption, and, on the other hand, a mixture D composed of the reaction products devoid of hydrogen and of inert gas;
v) distilling said mixture D of organic products, resulting in the recovery of the VF₃ at the column top and of a mixture E at the column bottom composed of unconverted CTFE and also reaction byproducts.

2. Process according to Claim 1, comprising an additional stage vi) of distillation of said mixture E on a second column in order to recover and recycle the unconverted CTFE at the column top and to remove the reaction byproducts at the bottom of this second column.

3. Process according to either of Claims 1 and 2, comprising an additional stage of storage of the VF₃ obtained in stage v) in the presence of an amount of limonene sufficient to stabilize it at a maximum temperature of 50°C.

4. Process according to one of Claims 1 to 3, in which said metal is deposited on an alumina or active charcoal support.

5. Process according to one of Claims 1 to 4, in which the catalyst is based on Pd deposited on an alumina support.

6. Process according to one of Claims 1 to 5, in which the solvent used in stage iii) is an alcohol comprising from 1 to 4 carbon atoms, preferably ethanol.

7. Process according to one of Claims 1 to 6, in which the drying of the mixture B after removing the hydracids is carried out over a molecular sieve, such as siliporite.

8. Process according to one of Claims 1 to 7, in which said inert gas is nitrogen, the nitrogen/H₂ molar ratio ranging from 0/1 to 2/1 and preferably from 0/1 to 1/1.

9. Process according to one of Claims 1 to 8, in which said reactor has a jacket filled with a heat-exchange fluid, the temperature of which is maintained at between 25 and 50°C.

10. Process for the preparation of a fluorinated copolymer or terpolymer comprising, as monomer, VF₃ and, as comonomer, at least one partially or completely fluorinated olefin, said process comprising the following stages of preparation of the VF₃ carried out at atmospheric pressure:
i) introducing, into a reactor, a gas mixture A composed of hydrogen, CTFE and optionally an inert gas over a bed of catalyst based on a metal from Group VIII deposited on a support, the H₂/CTFE molar ratio ranging from 0.5/1 to 2/1, resulting, after a sufficient contact time, in the achievement of a gas mixture B composed of the reaction products comprising VF₃ and organic byproducts, and also of unconsumed H₂, said inert gas and unconsumed CTFE and of hydracids;
ii) removing the hydracids present in the mixture B by washing with water, followed by washing with a dilute base and then drying, resulting in the recovery of a gas mixture C composed of reaction products comprising VF₃ and organic byproducts, and also of unconsumed H₂, said inert gas and of unconsumed CTFE;
iii) passing the gas mixture C through a countercurrentwise column of a solvent, at a temperature lower than ambient temperature, resulting in the achievement, on the one hand, of the hydrogen and the inert gas and, on the other hand, of a mixture composed of organic products dissolved in said solvent;
iv) desorbing, by heating to boiling point, the organic products dissolved in the solvent in order to obtain, on the one hand, the solvent, which will be recycled to the absorption, and, on the other hand, a mixture D composed of the reaction products devoid of hydrogen and of inert gas;
v) distilling said mixture D of organic products, resulting in the recovery of the VF₃ at the column top and of a mixture E at the column bottom composed of unconverted CTFE and also reaction byproducts.

11. Process according to Claim 10, in which said olefin is chosen from tetrafluoroethylene, chlorotrifluoroethylene, hexafluoropropylene, 1,1,3,3,3-pentafluoropropene, vinyl fluoride, vinylidene fluoride, 2,3,3,3-tetrafluoropropene and 1,1-chlorofluoroethylene.

12. Process according to either of Claims 10 and 11, resulting in the poly(VF₃/VF₂/CFE) terpolymer being obtained.

13. Process according to Claim 10, in which, said olefin being CTFE, the process results, at the end of stage v), in a mixture of VF₃ and CTFE being obtained at the distillation column top.
